# EUROPEAN PATENT APPLICATION

(11) **EP 4 851 281 A1**
(43) Date of publication of application: **09.12.2026**
(21) Application number: 25748169.7
(22) Date of filing: 28.01.2025
(51) Int. Cl.: C12N 9/26, C12N 1/15, C12N 1/19, C12N 1/21, C12N 5/10, C12N 15/09, C12N 15/31, C12N 15/56, C12N 15/63, C13B 50/00

(54) **MODIFIED TRANSGLUCOSIDASE**

(30) Priority: 29.01.2024 JP 2024011342
(71) Applicant: Amano Enzyme Inc., Nagoya-shi Aichi 460-8630 (JP)
(72) Inventor: YANG, Minghong, Kakamigahara-shi, Gifu 509-0109 (JP); OKUYAMA, Masayuki, Sapporo-shi, Hokkaido 060-8589 (JP); TAGAMI, Takayoshi, Sapporo-shi, Hokkaido 060-8589 (JP); TOTSUKA, Misaki, Sapporo-shi, Hokkaido 060-8589 (JP)
(74) Representative: J A Kemp LLP
(86) International application number: PCT/JP2025/002503
(87) International publication number: WO 2025/164587

(57) **Abstract**

The present invention aims to provide a transglucosidase having modified reaction specificity, as well as an enzyme preparation, a method for producing a saccharide-containing composition, and the like, using the modified transglucosidase. The above goal is achieved by providing a modified transglucosidase consisting of a polypeptide consisting of an amino acid sequence resulting from introduction of at least one of the substitutions: (A) substitution of the amino acid residue at position 227 with a leucine residue or a phenylalanine residue, (B) substitution of the amino acid residue at position 343 with a tyrosine residue, and (C) substitution of the amino acid residue at position 493 with a valine residue, into the amino acid sequence shown in SEQ ID NO: 1 or 13, and the like.

## Description

### [Technical Field]

The present invention relates to a transglucosidase having modified reaction specificity. The present invention also relates to use of the transglucosidase for various applications.

### [Background Art]

Transglucosidase (a-glucosidase) is an enzyme that mainly hydrolyzes the α-1,4-glucosidic bond at the non-reducing end of maltooligosaccharides, and when the substrate reaches a high concentration, catalyzes glycosyl transfer activity to produce isomaltooligosaccharides having α-1,6-glucosidic bonds. Transglucosidase is used for producing isomaltooligosaccharides using maltose produced by starch degradation as a raw material, and for use in improving flavor by adding during production of alcoholic beverages to produce oligosaccharides.

To date, various reports have been made regarding modification of transglucosidase.

For example, Patent Literature 1 describes that hydrolytic activity can be reduced to 1/10,000 or less by introducing a mutation into a transglucosidase derived from *Schizosaccharomyces pombe.*

Patent Literature 2 describes that glycosyl transfer activity toward a natural substrate can be improved by substituting the aspartic acid residue or glutamic acid residue of the nucleophilic catalytic residue of an anomer-retaining glycoside hydrolase with a cysteine sulfinic acid residue and/or a cysteine sulfonic acid residue.

Patent Literature 3 describes a method for converting a dextran glucosidase derived from *Streptococcus mutans* into a protein having reduced hydrolytic activity and enhanced glycosyl transfer activity by a protein engineering technique.

Patent Literature 4 describes a method for producing an α-anomer-selective glycoside by using a glycosyl transfer reaction by an α-glucosidase of the genus *Xanthomonas.*

Patent Literature 5 describes that glycosyl transfer activity is made predominant by introducing a mutation into an α-glucosidase derived from *Aspergillus niger.*

Non Patent Literature 1 describes that isomaltose and isomaltotriose can be accumulated in large amounts by introducing a mutation into an α-glucosidase derived from *Aspergillus niger.*

### [Citation List]

### [Patent Literature]

[Patent Literature 1]
   JP 2003-88365 A
[Patent Literature 2]
   JP 2005-253302 A
[Patent Literature 3]
   JP 2009-22204 A
[Patent Literature 4]
   JP 2001-046096 A
[Patent Literature 5]
   JP 2012-124520 A2

### [Non Patent Literature]

[Non Patent Literature 1]
Min Ma, et al., Applied microbiology and biotechnology, 101(16), 6399-6408, 2017

### [Summary of Invention]

### [Technical Problem]

However, further technical improvement is desired for producing various saccharides by transglucosidase.

Therefore, an object of the present invention is to provide a transglucosidase having modified reaction specificity. In addition, an object of the present invention is to provide an enzyme preparation, a method for producing a saccharide-containing composition, and the like, using the modified transglucosidase.

### [Solution to Problem]

The present inventors have conducted intensive studies in an attempt to solve the above-mentioned problems and successfully found a novel mutation capable of modifying reaction specificity in a transglucosidase derived from a microorganism of the genus *Aspergillus* (*Aspergillus niger*)*,* which resulted in the completion of the present invention. In view of the common general technical knowledge that enzymes of the same type are highly similar in structure (primary structure and three-dimensional structure) and similar mutations highly likely will produce similar effects, the mutation method found by the present inventors can be applied not only to the transglucosidase derived from *Aspergillus niger* shown in the Examples described later, but also to transglucosidases derived from other organisms.

That is, one aspect of the present invention relates to the following.
[1] A modified transglucosidase consisting of a polypeptide shown in any of the following (1) to (3):
   (1) a polypeptide consisting of an amino acid sequence resulting from introduction of at least one of the substitutions (A) to (C):
      (A) substitution of the amino acid residue at position 227 with a leucine residue or a phenylalanine residue,
      (B) substitution of the amino acid residue at position 343 with a tyrosine residue, and
      (C) substitution of the amino acid residue at position 493 with a valine residue
      into the amino acid sequence shown in SEQ ID NO: 1 or 13,
   (2) a polypeptide consisting of an amino acid sequence into which at least any one substitution shown in the aforementioned (A) to (C) has been introduced, wherein one or several amino acid residues other than the aforementioned substituted amino acid residue(s) are substituted, added, inserted, or deleted, and the polypeptide has modified reaction specificity as compared with the polypeptide consisting of the amino acid sequence shown in SEQ ID NO: 1 or 13, and
   (3) a polypeptide consisting of an amino acid sequence into which at least any one substitution shown in the aforementioned (A) to (C) has been introduced, wherein the sequence identity of the portion excluding the aforementioned substituted amino acid residue(s) is 70% or more, and the polypeptide has modified reaction specificity as compared with the polypeptide consisting of the amino acid sequence shown in SEQ ID NO: 1 or 13.
[2] A DNA encoding the modified transglucosidase of [1].
[3] An expression cassette or a recombinant vector comprising the DNA of [2].
[4] A transformant obtained by introducing the expression cassette or recombinant vector of [3] into a host.
[5] A method for producing a modified transglucosidase, comprising a step of culturing the transformant of [4].
[6] An enzyme preparation comprising the modified transglucosidase of [1].
[7] A method for producing a saccharide-containing composition, comprising a step of allowing the modified transglucosidase of [1] to act on a saccharide-containing raw material.
[8] The method of [7], wherein the saccharide-containing composition comprises at least any one selected from the group consisting of maltotriose, isomaltose, panose, centose, isomaltotriose, 3'-O-α-glucosylmaltose, kojibiose, and nigerose.
[9] The method of [7] or [8], wherein the saccharide-containing composition is a food or beverage.

### [Advantageous Effects of Invention]

According to the present invention, a transglucosidase having modified reaction specificity can be provided. Further, according to the present invention, an enzyme preparation, a method for producing a saccharide-containing composition, and the like can be provided using the modified transglucosidase.

### [Description of Embodiments]

An example of an embodiment of the present invention is described in detail below, but the present invention is not limited thereto. For convenience of explanation, some of the terms used in relation to the present invention are defined in the following.

### (Terms)

In the present specification, "transglucosidase" means a glucosidase represented by EC No. 3.2.1.20. Transglucosidase is also referred to as α-glucosidase. The transglucosidase is preferably derived from *Aspergillus niger.* The transglucosidase derived from *Aspergillus niger* can be obtained, for example, from Amano Enzyme Inc. In the present specification, the term "transglucosidase" and the term "α-glucosidase" are used interchangeably.

In the present specification, except in the Sequence Listing, 20 types of amino acid residues in an amino acid sequence may be represented by one-letter abbreviations. That is, glycine (Gly) is G, alanine (Ala) is A, valine (Val) is V, leucine (Leu) is L, isoleucine (Ile) is I, phenylalanine (Phe) is F, tyrosine (Tyr) is Y, tryptophan (Trp) is W, serine (Ser) is S, threonine (Thr) is T, cysteine (Cys) is C, methionine (Met) is M, aspartic acid (Asp) is D, glutamic acid (Glu) is E, asparagine (Asn) is N, glutamine (Gln) is Q, lysine (Lys) is K, arginine (Arg) is R, histidine (His) is H, and proline (Pro) is P. In the present specification, the amino acid at a mutation introduction site is represented by a combination of one letter representing the type of amino acid and a numeral representing the position of the amino acid. For example, when the tryptophan at position 343 is the mutation introduction site, it is represented as "W343".

In the present specification, in the amino acid sequence shown, the left end is the N-terminus and the right end is the C-terminus.

In the present specification, "non-polar amino acids" include alanine, valine, leucine, isoleucine, proline, methionine, phenylalanine, and tryptophan. "Uncharged amino acids" include glycine, serine, threonine, cysteine, tyrosine, asparagine, and glutamine. "Acidic amino acids" include aspartic acid and glutamic acid. "Basic amino acids" include lysine, arginine, and histidine.

In the present specification, "substitution" refers not only to cases where amino acid residue substitutions are artificially introduced, but also to cases where amino acid residue substitutions are naturally introduced, i.e., cases where the amino acid residues were originally different. In the present specification, the substitution of amino acid residues may be either artificial or natural, but artificial substitution is preferred.

In the present specification, "reaction specificity" refers to a property of producing a specific product when a specific enzyme is allowed to act on a specific substrate. "Modification of reaction specificity" refers to modifying the property of an enzyme so as to produce different products from the same substrate, and/or modifying the property of an enzyme such that the production ratio of a plurality of products produced from the same substrate changes. The modified transglucosidase of the present invention has modified reaction specificity. The reaction specificity of the modified transglucosidase of the present invention is not particularly limited as long as it is modified relative to the original transglucosidase. Examples thereof include the modified transglucosidase of the present invention in which reactivity to an α-1,6 bond (transfer activity and/or degradation activity), reactivity to an α-1,2 bond (transfer activity and/or degradation activity), or reactivity to an α-1,3 bond (transfer activity and/or degradation activity) is improved. For example, the property is modified such that the production ratio of at least any one of maltotriose, isomaltose, panose, centose, isomaltotriose, 3'-O-α-glucosylmaltose, kojibiose, and nigerose increases. Preferably, the property is modified such that the production ratio of maltotriose and centose increases (mutant 1), the production ratio of maltotriose, isomaltose, centose, kojibiose, and nigerose increases (mutant 2), the production ratio of maltotriose, centose, 3'-O-glucosylmaltose, kojibiose, and nigerose increases (mutant 3), the production ratio of panose increases (mutant 4), or the production ratio of 3'-O-glucosylmaltose, kojibiose, and nigerose increases (mutant 5).

Maltotriose, isomaltose, panose, centose, isomaltotriose, 3'-O-α-glucosylmaltose, kojibiose, and nigerose each have the structures shown below.
·Maltotriose: Glc-α-1,4-Glc-α-1,4-Glc
·Isomaltose: Glc-α-1,6-Glc
·Panose: Glc-α-1,6-Glc-α-1,4-Glc
·Centose: Glc-α-1,2-Glc-α-1,4-Glc
· Isomaltotriose: Glc-α-1,6-Glc-α-1,6-Glc
·3'-Q-glucosylmaltose: Glc-α-1,3-Glc-α-1,4-Glc
·Kojibiose: Glc-α-1,2-Glc
·Nigerose: Glc-α-1,3-Glc

### (Modified Transglucosidase)

The modified transglucosidase of the present invention consists of a polypeptide shown in any of the following (1) to (3) :
(1) a polypeptide consisting of an amino acid sequence resulting from introduction of at least one of the substitutions shown in (A) to (C):
   (A) substitution of the amino acid residue at position 227 with a leucine residue or a phenylalanine residue,
   (B) substitution of the amino acid residue at position 343 with a tyrosine residue, and
   (C) substitution of the amino acid residue at position 493 with a valine residue
   into the amino acid sequence shown in SEQ ID NO: 1 or 13,
(2) a polypeptide consisting of an amino acid sequence into which at least any one substitution shown in the aforementioned (A) to (C) has been introduced, wherein one or several amino acid residues other than the aforementioned substituted amino acid residue(s) are substituted, added, inserted, or deleted, and the polypeptide has modified reaction specificity as compared with the polypeptide consisting of the amino acid sequence shown in SEQ ID NO: 1 or 13, and
(3) a polypeptide consisting of an amino acid sequence into which at least any one substitution shown in the aforementioned (A) to (C) has been introduced, wherein the sequence identity of the portion excluding the aforementioned substituted amino acid residue(s) is 70% or more, and the polypeptide has modified reaction specificity as compared with the polypeptide consisting of the amino acid sequence shown in SEQ ID NO: 1 or 13.

The polypeptide of the aforementioned (1) is more specifically as follows.
(a) a polypeptide consisting of an amino acid sequence resulting from introduction of substitution of the amino acid residue at position 227 with a leucine residue or a phenylalanine residue in the amino acid sequence shown in SEQ ID NO: 1
(b) a polypeptide consisting of an amino acid sequence resulting from introduction of substitution of the amino acid residue at position 343 with a tyrosine residue in the amino acid sequence shown in SEQ ID NO: 1
(c) a polypeptide consisting of an amino acid sequence resulting from introduction of substitution of the amino acid residue at position 493 with a valine residue in the amino acid sequence shown in SEQ ID NO: 1
(d) a polypeptide consisting of an amino acid sequence resulting from introduction of substitution of the amino acid residue at position 227 with a leucine residue or a phenylalanine residue, and substitution of the amino acid residue at position 343 with a tyrosine residue in the amino acid sequence shown in SEQ ID NO: 1
(e) a polypeptide consisting of an amino acid sequence resulting from introduction of substitution of the amino acid residue at position 227 with a leucine residue or a phenylalanine residue, and substitution of the amino acid residue at position 493 with a valine residue in the amino acid sequence shown in SEQ ID NO: 1
(f) a polypeptide consisting of an amino acid sequence resulting from introduction of substitution of the amino acid residue at position 343 with a tyrosine residue, and substitution of the amino acid residue at position 493 with a valine residue in the amino acid sequence shown in SEQ ID NO: 1
(g) a polypeptide consisting of an amino acid sequence resulting from introduction of substitution of the amino acid residue at position 227 with a leucine residue or a phenylalanine residue, substitution of the amino acid residue at position 343 with a tyrosine residue, and substitution of the amino acid residue at position 493 with a valine residue in the amino acid sequence shown in SEQ ID NO: 1
(h) a polypeptide consisting of an amino acid sequence resulting from introduction of substitution of the amino acid residue at position 227 with a leucine residue or a phenylalanine residue in the amino acid sequence shown in SEQ ID NO: 13
(i) a polypeptide consisting of an amino acid sequence resulting from introduction of substitution of the amino acid residue at position 343 with a tyrosine residue in the amino acid sequence shown in SEQ ID NO: 13
(j) a polypeptide consisting of an amino acid sequence resulting from introduction of substitution of the amino acid residue at position 493 with a valine residue in the amino acid sequence shown in SEQ ID NO: 13
(k) a polypeptide consisting of an amino acid sequence resulting from introduction of substitution of the amino acid residue at position 227 with a leucine residue or a phenylalanine residue, and substitution of the amino acid residue at position 343 with a tyrosine residue in the amino acid sequence shown in SEQ ID NO: 13
(l) a polypeptide consisting of an amino acid sequence resulting from introduction of substitution of the amino acid residue at position 227 with a leucine residue or a phenylalanine residue, and substitution of the amino acid residue at position 493 with a valine residue in the amino acid sequence shown in SEQ ID NO: 13
(m) a polypeptide consisting of an amino acid sequence resulting from introduction of substitution of the amino acid residue at position 343 with a tyrosine residue, and substitution of the amino acid residue at position 493 with a valine residue in the amino acid sequence shown in SEQ ID NO: 13
(n) a polypeptide consisting of an amino acid sequence resulting from introduction of substitution of the amino acid residue at position 227 with a leucine residue or a phenylalanine residue, substitution of the amino acid residue at position 343 with a tyrosine residue, and substitution of the amino acid residue at position 493 with a valine residue in the amino acid sequence shown in SEQ ID NO: 13

In the polypeptide of the aforementioned (2), the modification of amino acid to be introduced may include only one kind of modification selected from substitution, addition, insertion, and deletion (for example, substitution only), or may include two or more kinds of modifications (for example, substitution and insertion). In the polypeptide of the aforementioned (2), the number of amino acid differences at optional different sites may be one or several, and examples thereof include 1 to 80, preferably 1 to 70, 1 to 60, 1 to 50, 1 to 40, or 1 to 30, more preferably 1 to 20, 1 to 10, 1 to 8, 1 to 7, 1 to 6, 1 to 5, or 1 to 4, further preferably 1 to 3, particularly preferably 1 or 2, or 1.

In the polypeptide of the aforementioned (3), the sequence identity to the amino acid sequence shown in SEQ ID NO: 1 or 13 may be 70% or more, preferably 75% or more, more preferably 80% or more, further preferably 85% or more, further more preferably 90% or more, particularly preferably 93% or more, 95% or more, 97% or more, 98% or more, or 99% or more.

Here, in the polypeptide of the aforementioned (3), the sequence identity to each amino acid sequence shown in SEQ ID NO: 1 or 13 is sequence identity calculated in comparison with the amino acid sequence shown in SEQ ID NO: 1 or 13. The "sequence identity" refers to the value of amino acid sequence identity obtained by the bl2seq program (Tatiana A. Tatsusova, Thomas L. Madden, FEMS Microbiol. Lett., Vol. 174, p. 247-250, 1999) of BLASTPACKAGE [sgi32 bit edition, Version 2.0.12; available from National Center for Biotechnology Information (NCBI)]. The parameters may be set as follows: Gap insertion Cost value: 11, Gap extension Cost value: 1.

In the polypeptides of the aforementioned (2) and (3), amino acid residues corresponding to position 490 (aspartic acid residue) and position 660 (aspartic acid residue) of the amino acid sequence shown in SEQ ID NO: 1 or 13 are considered to be active catalytic residues, and therefore it is desirable not to introduce substitution or deletion into these sites.

The polypeptide of the present invention may be part of a larger protein (for example, a fusion protein). Examples of sequences to be added in the fusion protein include sequences useful for purification, such as multiple histidine residues, additional sequences that ensure stability during recombinant production, and the like.

In the polypeptides of the aforementioned (2) and (3), when an amino acid substitution is introduced into SEQ ID NO: 1 or 13, a conservative substitution is mentioned as a preferred embodiment of the amino acid substitution to be introduced. That is, examples of the substitution in the polypeptides of the aforementioned (2) and (3) include substitution with another nonpolar amino acid when the amino acid before substitution is a nonpolar amino acid, substitution with another uncharged amino acid when the amino acid before substitution is an uncharged amino acid, substitution with another acidic amino acid when the amino acid before substitution is an acidic amino acid, and substitution with another basic amino acid when the amino acid before substitution is a basic amino acid.

The polypeptides of the aforementioned (2) and (3) have α-glucosidase activity and have modified reaction specificity. The modified reaction specificity refers to a characteristic of producing a product having a composition different from that of the polypeptide consisting of the amino acid sequence shown in SEQ ID NO: 1 or 13 when allowed to act on the same substrate. Specifically, it is a characteristic in which the production ratio of panose or centose increases when maltose is used as a substrate, and means that the production amount of panose or centose is 1.1 times or more. It is preferably 1.2 times or more, more preferably 1.3 times or more. As another embodiment, a characteristic in which the production ratio of isomaltose increases when maltose is used as a substrate is mentioned. The production amount of isomaltose is, for example, 1.2 times, preferably 1.5 times or more or 2 times or more.

**[Table 1]**

| SEQ ID NO: | explanation |
|---|---|
| 1 | reference sequence 1 (amino acid sequence) |
| 2 | nucleotide sequence of SEQ ID NO: 1 |
| 3 | G227L mutation in SEQ ID NO: 1 |
| 4 | G227F mutation in SEQ ID NO: 1 |
| 5 | G227F/W343Y mutation in SEQ ID NO: 1 |
| 6 | E493V mutation in SEQ ID NO: 1 |
| 7 | W343Y mutation in SEQ ID NO: 1 |
| 8 | nucleotide sequence of SEQ ID NO: 3 |
| 9 | nucleotide sequence of SEQ ID NO: 4 |
| 10 | nucleotide sequence of SEQ ID NO: 5 |
| 11 | nucleotide sequence of SEQ ID NO: 6 |
| 12 | nucleotide sequence of SEQ ID NO: 7 |
| 13 | reference sequence 2 (amino acid sequence) |
| 14 | nucleotide sequence of SEQ ID NO: 13 |
| 15 | G227L mutation in SEQ ID NO: 13 |
| 16 | G227F mutation in SEQ ID NO: 13 |
| 17 | G227F/W343Y mutation in SEQ ID NO: 13 |
| 18 | E493V mutation in SEQ ID NO: 13 |
| 19 | W343Y mutation in SEQ ID NO: 13 |
| 20 | nucleotide sequence of SEQ ID NO: 15 |
| 21 | nucleotide sequence of SEQ ID NO: 16 |
| 22 | nucleotide sequence of SEQ ID NO: 17 |
| 23 | nucleotide sequence of SEQ ID NO: 18 |
| 24 | nucleotide sequence of SEQ ID NO: 19 |

Each mutation in the modified transglucosidase of the present invention has, for example, the following advantageous properties as compared with a reference enzyme (for example, an enzyme consisting of the amino acid sequence shown in SEQ ID NO: 1 or 13), but is not limited thereto.
·G227L: increases in α-(1→4) transfer and α-(1→2) transfer
·G227F: increases in α-(1→4) transfer, α-(1→2) transfer, and α- (1→3) transfer
·G227F/W343Y: increases in α-(1→4) transfer, α-(1→2) transfer, and α-(1→3) transfer, and decrease in α-(1→6) transfer
·E493V: increase in α-(1→6) transfer at pH 2.5
·W343Y: increases in α-(1→2) transfer and α-(1→3) transfer

The enzyme to be mutated in the present invention is transglucosidase. The enzyme to be mutated is typically a wild-type enzyme (an enzyme found in nature). However, an enzyme to which some mutation or modification has already been applied may be used as the enzyme to be mutated. The enzyme to be mutated is not particularly limited, and examples thereof include human maltase-glucoamylase, *Aspergillus niger* alpha-glucosidase, human neutral alpha-glucosidase C, mouse lysosomal alpha-glucosidase, yeast alpha-glucosidase (Yeast GLU2A), *Aspergillus nidulans* alpha-glucosidase AgdA, *Aspergillus nidulans* alpha-glucosidase AgdB, *Mucor javanicus* alpha-glucosidase, *Aspergillus oryzae* alpha-glucosidase, *Mortierella alliacea* alpha-glucosidase, *Schizosaccharomyces pombe* alpha-glucosidase, *Debaryomyces occidentalis* alpha-glucosidase, barley alpha-glucosidase *(Hordeum vulgare* subsp. *vulgare* alpha-glucosidase), *Arabidopsis thaliana* alpha-glucosidase, spinach alpha-glucosidase *(Spinacia oleracea* alpha-glucosidase), sugar beet alpha-glucosidase *(Beta vulgaris* alpha-glucosidase), potato alpha-glucosidase *(Solanum tuberosum* alpha-glucosidase), and the like. Preferably, it is a transglucosidase derived from *Aspergillus niger.*

### (DNA)

The DNA of the present invention is DNA encoding the polypeptide described above. The DNA of the present invention is not particularly limited as long as it is DNA having a nucleotide sequence encoding a modified transglucosidase consisting of the polypeptide shown in (1) to (3) described above. The nucleotide sequence shown in SEQ ID NO: 2 is the cDNA sequence of a gene encoding a polypeptide consisting of the amino acid sequence shown in SEQ ID NO: 1 (at times referred to as "reference sequence 1"). Therefore, the DNA of the present invention can be appropriately designed by a person skilled in the art by using SEQ ID NO: 2 as a reference sequence. In addition, the nucleotide sequence shown in SEQ ID NO: 14 is the cDNA sequence of a gene encoding a polypeptide consisting of the amino acid sequence shown in SEQ ID NO: 13 (at times referred to as "reference sequence 2"). Therefore, the DNA of the present invention can be appropriately designed by a person skilled in the art by using SEQ ID NO: 14 as a reference sequence. SEQ ID NO: 1 has alanine at position 694, and SEQ ID NO: 13 has asparagine at position 694.

Examples of the DNA of the present invention include DNA shown in any of the following (i) to (iii):
(i) DNA consisting of a nucleotide sequence resulting from introduction of at least one of the substitutions (a) to (c): (a) substitution with a nucleotide sequence encoding a leucine residue or a phenylalanine residue at positions 679 to 681, (b) substitution with a nucleotide sequence encoding a tyrosine residue at positions 1027 to 1029, (c) substitution with a nucleotide sequence encoding a valine residue at positions 1477 to 1479 in the nucleotide sequence shown in SEQ ID NO: 2 or 14,
(ii) DNA encoding a polypeptide having modified reaction specificity as compared with the polypeptide consisting of the amino acid sequence shown in SEQ ID NO: 1 or 13, the DNA hybridizing under stringent conditions with DNA consisting of a nucleotide sequence complementary to the DNA shown in the above-mentioned (i), and
(iii) DNA encoding a polypeptide having modified reaction specificity as compared with the polypeptide consisting of the amino acid sequence shown in SEQ ID NO: 1 or 13, the DNA having 70% or more homology with the DNA shown in the above-mentioned (i) .

The DNA of the present invention is not limited to the above-mentioned sequences, and may be one having 70% or more homology with the nucleotide sequences shown in SEQ ID NOs: 8 to 12 and 20 to 24. DNA having a nucleotide sequence having preferably 75% or more, more preferably 80% or more, further preferably 85% or more, further more preferably 90% or more, particularly preferably 93% or more, 95% or more, 97% or more, 98% or more, or 99% or more homology is also included in the DNA of the present invention, as long as it encodes a polypeptide having transglucosidase activity.

Here, the "homology" of DNA is calculated using publicly available or commercially available software with an algorithm for comparing a reference sequence with a query sequence. Specifically, BLAST, FASTA, or GENETYX (Software Development Co., Ltd.) can be used, and these may be used with default parameters. "Homology" can be reworded as "sequence identity".

Corresponding to the above-described substitution, addition, insertion, or deletion of the amino acid sequence, a nucleotide sequence in which mutations of substitution, addition, insertion, or deletion have occurred in several nucleotides in the nucleotide sequences described in SEQ ID NOs: 8 to 12 and 20 to 24 is also included in the DNA of the present invention, as long as it encodes a polypeptide having transglucosidase activity.

Furthermore, DNA that hybridizes under stringent conditions with DNA consisting of a nucleotide sequence complementary to DNA consisting of the nucleotide sequences described in SEQ ID NOs: 8 to 12 and 20 to 24 is also included in the DNA of the present invention, as long as it encodes a polypeptide having transglucosidase activity.

Here, "under stringent conditions" refers to conditions such as 4 hours to overnight incubation at 50°C to 65°C in 0.5% SDS, 5x Denhartz's [0.1% bovine serum albumin (BSA), 0.1% polyvinylpyrrolidone, 0.1% Ficoll 400] and 6x SSC containing 100 µg/ml salmon sperm DNA (1x SSC is 0.15M NaCl and 0.015M sodium citrate, pH 7.0).

Hybridization under stringent conditions is specifically performed by the following method. That is, a nylon membrane with a DNA library or cDNA library immobilized is prepared, and the nylon membrane is blocked in a prehybridization solution containing 6xSSC, 0.5% SDS, 5xDenhartz's, and 100 µg/ml salmon sperm DNA at 65°C. Then, each probe labeled with 32P is added and incubated at 65°C overnight. The nylon membrane is washed in 6xSSC at room temperature for 10 minutes, in 2xSSC containing 0.1% SDS at room temperature for 10 minutes, and in 0.2xSSC containing 0.1% SDS at 45°C for 30 minutes, and then autoradiography is performed to detect DNA that has specifically hybridized with the probe.

The DNA of the present invention can also be isolated from a microorganism producing the above-mentioned predetermined polypeptide. For example, using genomic DNA of *Aspergillus niger* as a template, the target DNA can be isolated from the genome of the aforementioned microorganism by PCR or a hybridization method using primers or probes designed in consideration of gene degeneracy from known amino acid sequence information, or primers or probes designed based on known nucleotide sequence information.

The DNA of the present invention includes various DNAs derived from codon degeneracy. Artificially producing various DNAs encoding the same amino acid sequence can be easily performed using known genetic engineering techniques. For example, in production of a genetic-engineering protein, when a codon used on the original gene encoding the target protein has a low frequency of use in a host, the expression amount of the protein may be low. In such a case, high expression of the target protein can be achieved by optimizing the codon usage frequency to the host, without changing the encoded amino acid sequence.

As an index showing the codon usage frequency, the total of host optimal codon usage frequencies of each codon may be adopted. The optimal codon is defined as the codon having the highest frequency of use among codons corresponding to the same amino acid. The codon usage frequency is not particularly limited as long as it is optimized to the host, and examples of optimal codons of *Escherichia coli* include the following. F: phenylalanine (ttt), L: leucine (ctg), I: isoleucine (att), M: methionine (atg), V: valine (gtg), Y: tyrosine (tat), stop codon (taa), H: histidine (cat), Q: glutamine (cag), N: asparagine (aat), K: lysine (aaa), D: aspartic acid (gat), E: glutamic acid (gaa), S: serine (agc), P: proline (ccg), T: threonine (acc), A: alanine (gcg), C: cysteine (tgc), W: tryptophan (tgg), R: arginine (cgc), and G: glycine (ggc).

As a method for introducing a mutation into a gene and artificially modifying an amino acid sequence, known methods such as the Kunkel method and the Gapped duplex method, and mutation introduction kits using site-directed mutagenesis, for example, QuikChange^{™} Site-Directed Mutagenesis Kit (Stratagene), GeneTailor (trademark) Site-Directed Mutagenesis System (Invitrogen), TaKaRa Site-Directed Mutagenesis System (Mutan-K, Mutan-Super Express Km, etc.; Takara Bio Inc.), and the like can be used.

Confirmation of the nucleotide sequence of DNA can be performed by sequencing by a conventional method. For example, it can be performed by the dideoxynucleotide chain termination method (Sanger et al. (1977) Proc. Natl. Acad. Sci. USA 74:5463), and the like. In addition, the sequence can be analyzed using an appropriate DNA sequencer.

Confirmation as to whether the obtained DNA is DNA encoding the target polypeptide can be performed by comparing the determined nucleotide sequence with the nucleotide sequence described in SEQ ID NO: 2. Alternatively, it can be performed by comparing the amino acid sequence deduced from the determined nucleotide sequence with the amino acid sequence described in SEQ ID NO: 1 or 13.

### (Expression Cassette or Recombinant Vector)

An expression cassette or recombinant vector comprising DNA encoding the above-mentioned predetermined polypeptide (hereinafter also referred to as "the expression cassette of the present invention" or "the recombinant vector of the present invention") can be obtained by linking a promoter and a terminator to the DNA of the present invention, or by inserting the expression cassette or the DNA of the present invention into an expression vector.

The expression cassette or recombinant vector of the present invention may further contain, as control factors, transcription elements such as an enhancer, a CCAAT box, a TATA box, and an SPI site, as necessary, in addition to a promoter and a terminator. These control factors may be operably linked to the DNA of the present invention. Operably linked means that various control factors that regulate the DNA of the present invention are linked to the DNA of the present invention in a state in which they can operate in a host cell.

With respect to the recombinant vector of the present invention, as the expression vector, a vector constructed for gene recombination from a phage, a plasmid, or a virus capable of autonomously replicating in a host is preferred. Such expression vectors are well known, and examples of commercially available expression vectors include pQE series vectors (Qiagen Co., Ltd.), pDR540, pRIT2T (GE Healthcare Biosciences), pET vectors (Merck), pUC-series vectors (Takara Bio Inc.), and the like. The expression vector can be used in an appropriate combination with the host cell. For example, when *E*. *coli* is used as the host cell, examples of suitable combinations include a combination of a pET vector and a DH5α *E. coli* strain, a combination of a pET vector and a BL21 (DE3) *E. coli* strain, or a combination of a pDR540 vector and a JM109 *E. coli* strain.

### (Transformant)

A transformant can be obtained by transforming a host with the expression cassette or recombinant vector of the present invention (hereinafter also referred to as "the transformant of the present invention").

The host used for producing a transformant is not particularly limited as long as gene introduction is possible, the expression cassette or recombinant vector is stable, and it is capable of autonomous growth and capable of expressing the trait of a gene comprising the DNA of the present invention. Preferred examples thereof include bacteria belonging to the genus *Escherichia,* such as *Escherichia coli,* the genus *Bacillus,* such as *Bacillus subtilis,* the genus *Pseudomonas,* such as *Pseudomonas putida,* and the like; filamentous fungi such as the genus *Aspergillus,* such as *Aspergillus oryzae;* yeasts; and the like. In addition, animal cells, insect cells, plants, and the like may be used.

The transformant of the present invention can be obtained by introducing the expression cassette of the present invention or the recombinant vector of the present invention into a host. The site of introduction of the DNA of the present invention and the like is not particularly limited as long as the target gene can be expressed, and may be on a plasmid or on a genome. Specific methods for introducing the expression cassette of the present invention or the recombinant vector of the present invention include, for example, the recombinant vector method and the genome editing method. The conditions for introducing the expression cassette or the recombinant vector into a host may be appropriately set according to the type of the host, etc. When the host is a bacterium, for example, examples of the methods including a method using calcium ion-treated competent cells, and the electroporation method. When the host is a filamentous fungus, examples of the methods include the protoplast-PEG method. When the host is a yeast, examples of the methods including the electroporation method, the spheroblast method, and the lithium acetate method. When the host is an animal cell, examples of the methods of the electroporation method, the calcium phosphate method, and the lipofection method. When the host is an insect cell, examples of the methods of the electroporation method, the calcium phosphate method, and the lipofection method. When the host is a plant cell, examples of the methods of the electroporation method, the agrobacterium method, the particle gun method, and the PEG method.

Whether or not the expression cassette of the present invention or the recombinant vector of the present invention has been incorporated into the host can be confirmed by PCR, Southern hybridization, Northern hybridization and the like.

When confirming whether the expression cassette or recombinant vector of the present invention has been incorporated into the host by PCR, for example, the genomic DNA or the expression cassette or recombinant vector may be isolated and purified from the transformant.

When the host is, for example, a bacterium, the isolation and purification of the expression cassette or recombinant vector may be carried out based on the lysate obtained by lysing the bacterium. For example, the lysis may be carried out by treating the bacterium with a lytic enzyme such as lysozyme, and, if necessary, protease and other enzymes as well as a surfactant such as sodium lauryl sulfate (SDS) may be used in combination.

Furthermore, physical disruption methods such as freeze-thaw and French press treatment may be combined. Isolation and purification of DNA from the lysate can be performed, for example, by an appropriate combination of deproteinization treatment using phenol treatment and protease treatment, ribonuclease treatment, alcohol precipitation treatment, and commercially available kits.

Cleavage of DNA can be performed according to standard methods, for example, using restriction enzyme treatment. For example, type II restriction enzymes that act on specific nucleotide sequences are used as restriction enzymes. Linkage of DNA to expression cassettes or expression vectors is performed, for example, using DNA ligase.

Then, using the isolated and purified DNA as a template, a primer specific to the DNA of the present invention is designed and PCR is performed. The PCR-obtained amplification product is subjected to agarose gel electrophoresis, polyacrylamide gel electrophoresis, capillary electrophoresis, etc., and stained with ethidium bromide and SYBR Green solution, etc., and the amplification product is detected as a band, so that transformation can be confirmed.

PCR can also be performed using a primer previously labeled with a fluorescent dye, etc., to detect the amplification product. Furthermore, a method of binding the amplification product to a solid phase such as a microplate and confirming the amplification product by fluorescence and enzyme reactions, etc., can also be adopted.

### (Method for Producing Enzyme)

The method for producing the modified transglucosidase of the present invention comprises a step of culturing the transformant of the present invention. When the substitution shown in at least one of (A) to (C) included in the modified transglucosidase is one that has been naturally introduced, the modified transglucosidase can be obtained by a production method comprising a step of culturing a microorganism producing the modified transglucosidase, without performing transformation using the expression cassette or recombinant vector of the present invention.

The above-mentioned culture conditions may be appropriately set in consideration of the nutritional and physiological properties of the above-mentioned transformant or the above-mentioned microorganism, and preferably include liquid culture. For industrial production, aeration and agitation culture is preferred. As the nutrient source for the medium, any substance required for the growth of the above-mentioned transformant or the above-mentioned microorganism may be used. As the carbon source, any assimilable carbon compound may be used, such as glucose, sucrose, lactose, maltose, molasses, pyruvic acid, etc. As the nitrogen source, any assimilable nitrogen compound may be used, such as peptone, meat extract, yeast extract, casein hydrolysate, and soybean meal alkali extract. In addition to the carbon and nitrogen sources, for example, salts such as phosphates, carbonates, sulfates, magnesium, calcium, potassium, iron, manganese, and zinc, specific amino acids, and specific vitamins may be used as necessary.

The culture temperature can be appropriately set within a range in which the above-mentioned transformant or the above-mentioned microorganism of the present invention can grow and the above-mentioned transformant or the above-mentioned microorganism produces the modified transglucosidase, and is preferably about 15 to 37°C. The culture may be completed at an appropriate time by estimating the time when the modified transglucosidase reaches the highest yield, and can be appropriately set. Generally, the culture time is about 12 to 120 hr.

After culturing the above-mentioned transformant or the above-mentioned microorganism, the culture supernatant or cells are recovered from the culture liquid by a method such as centrifugation, and the cells are treated by a mechanical method such as ultrasonication and French press or by a lytic enzyme such as lysozyme, and are solubilized, as necessary, by using an enzyme such as protease or a surfactant such as sodium lauryl sulfate (SDS), whereby a water-soluble fraction comprising the modified transglucosidase can be obtained. That is, in one embodiment of the present invention, the method for producing the modified transglucosidase of the present invention comprises a step of recovering the aforementioned cultured transformant. Further, in one embodiment of the present invention, the method comprises a step of obtaining the modified transglucosidase from the aforementioned recovered transformant.

In addition, the expressed modified transglucosidase can also be secreted into the culture liquid by selecting an appropriate expression cassette or expression vector and host.

The water-soluble fraction comprising the modified transglucosidase obtained as described above may be subjected as it is to purification treatment, or may be subjected to purification treatment after concentrating the modified transglucosidase in the water-soluble fraction.

Concentration can be performed, for example, by vacuum concentration, membrane concentration, salting out treatment, fractional precipitation using a hydrophilic organic solvent (e.g., methanol, ethanol, and acetone), etc.

Purification treatment of the above-specified modified transglucosidase can be performed by appropriately combining methods such as gel filtration, adsorption chromatography, ion exchange chromatography, and affinity chromatography.

The modified transglucosidase thus purified can be powdered by freeze drying, vacuum drying, spray drying, etc., as necessary, and distributed on the market.

### (Enzyme Preparation)

The modified transglucosidase can be provided in the form of an enzyme preparation. Therefore, the present invention also provides an enzyme preparation comprising the modified transglucosidase as an active ingredient.

The content of the modified transglucosidase in the enzyme preparation of the present invention is not particularly limited. The lower limit of the content is, for example, 0.01 U/g or more, preferably 0.1 U/g or more, more preferably 1 U/g or more, further preferably 10 U/g or more, or particularly preferably 100 U/g or more. The upper limit of the content is, for example, 1,000,000 U/g or less, preferably 500,000 U/g or less, 100,000 U/g or less, 50,000 U/g or less, or 10,000 U/g or less.

The enzyme preparation of the present invention may comprise components other than the above-mentioned predetermined modified transglucosidase to an extent that does not affect the effects of the present invention. Examples of other components include enzymes other than the above-mentioned predetermined modified transglucosidase, additives, culture residues produced in the above-mentioned production method, and the like.

Other enzymes include, for example, amylase (α-amylase, β-amylase, glucoamylase), glucosidase (β-glucosidase), galactosidase (α-galactosidase, β-galactosidase), protease (acid protease, neutral protease, alkaline protease), peptidase (leucine peptidase, aminopeptidase), lipase, esterase, cellulase, phosphatase (acid phosphatase, alkaline phosphatase), nuclease, deaminase, oxidase, dehydrogenase, glutaminase, pectinase, catalase, dextranase, transglutaminase, protein deamidase, pullulanase, and the like. These other enzymes may be contained alone or in combination of multiple types.

Additives include excipients, buffers, suspending agents, stabilizers, preservatives, antiseptics, physiological saline, and the like. Excipients include starch, dextrin, maltose, trehalose, lactose, D-glucose, sorbitol, D-mannitol, sucrose, glycerol, and the like. Buffers include phosphates, citrates, acetates, and the like. Stabilizers include propylene glycol, ascorbic acid, and the like. Preservatives include phenol, benzalkonium chloride, benzyl alcohol, chlorobutanol, methylparaben, and the like. Antiseptics include ethanol, benzalkonium chloride, paraoxybenzoic acid, chlorobutanol, sodium chloride, and the like. These additives may be contained alone or in combination.

Examples of culture residues include components derived from the medium, impurity proteins, microbial cell components, and the like.

The form of the enzyme preparation of the present invention is not particularly limited, and examples include liquid and solid forms (powder, granules, etc.) and the like. The aforementioned enzyme preparation can be prepared by a generally known method.

### (Use of Enzyme)

According to the present invention, a method for producing a saccharide-containing composition in which the sugar composition has changed is provided. Specifically, methods are provided for producing a saccharide-containing composition having an increased panose content ratio, a saccharide-containing composition having an increased centose content ratio, a saccharide-containing composition having an increased isomaltose content ratio, a saccharide-containing composition having an increased maltotriose content ratio, a saccharide-containing composition having an increased kojibiose content ratio, a saccharide-containing composition having an increased nigerose content ratio, and a saccharide-containing composition having an increased 3'-O-α-glucosylmaltose content ratio.

The method for producing a saccharide-containing composition of the present invention is characterized by comprising a step of allowing a modified transglucosidase to act on a saccharide-containing raw material. This makes it possible to change the sugar composition of the obtained saccharide-containing composition. A preferred embodiment is a method for producing a saccharide-containing composition having an increased content of maltotriose, isomaltose, panose, centose, isomaltotriose, 3'-O-α-glucosylmaltose, kojibiose, or nigerose, and a more preferred embodiment is a method for producing a saccharide-containing composition having an increased content of panose, centose, or isomaltose.

The saccharide-containing raw material used in the present invention is not particularly limited as long as it comprises a saccharide. For example, the type of saccharide may be starch, dextrin, or oligosaccharide, and may be maltose. It may be a single saccharide, or may comprise a plurality of types of saccharides. It may comprise only saccharide, or may comprise components other than saccharides (proteins, fats and oils, dietary fibers, inorganic salts, and the like). Examples of the saccharide-containing raw material comprising components other than saccharides include plant raw materials (legumes such as soy beans, green peas, lentils, chickpeas, black beans, broad beans, mung beans, lupin beans, kidney beans, and the like; cereals such as wheat, barley, oats, rice, rye, buckwheat, Japanese barnyard millet, foxtail millet, teff, and the like; nuts such as almonds, coconuts, peanuts, cashew nuts, hazelnuts, pecan nuts, macadamia nuts, pistachios, walnuts, Brazil nuts, pili nuts, chestnuts, sesame, pine nuts, and the like; hemp seeds (industrial hemp), chia seeds (Chia), quinoa (Quinoa), amaranth (Amaranthus), canary seeds, flaxseed, and the like), and the like. The shape of the saccharide-containing raw material is not particularly limited. Examples thereof include solid, liquid, and slurry forms, and liquid and slurry forms are preferred.

The use amount of the modified transglucosidase is not particularly limited, and the use amount per 1 g of saccharide is, for example, 0.0001 mg or more. Preferably, it is 0.001 mg or more or 0.005 mg or more, more preferably 0.01 mg or more, further preferably 0.05 mg or more. The upper limit of the use amount range of the modified transglucosidase per 1 g of saccharide is not particularly limited, and it is, for example, 1 g or less, 0.5 g or less, 0.1 g or less, 0.05 g or less, 0.01 g or less, or 0.001 g or less.

Regarding the hydrolytic activity of transglucosidase, the amount of enzyme that produces 1 µg of glucose per 1 minute by using α-methyl-D-glucoside as a substrate is defined as 1 unit (1 U).

In the present invention, for the purpose of further improving the effect, α-amylase can be used in combination with the above-mentioned modified transglucosidase. The origin of α-amylase is not particularly limited, and examples thereof include α-amylases derived from the genus *Aspergillus,* such as *Aspergillus oryzae, Aspergillus niger,* and the like, and the genus *Bacillus,* such as *Bacillus amyloliquefaciens, Bacillus subtilis, Bacillus licheniformis,* and the like, preferably α-amylase derived from the genus *Aspergillus,* more preferably α-amylase derived from *Aspergillus oryzae.*

Regarding the use amount of α-amylase, it is, for example, 0.01 to 100 U, preferably 0.05 to 50 U, more preferably 0.1 to 10 U, further preferably 0.3 to 3 U, per 1 g of a plant protein raw material.

Regarding the activity of α-amylase, the amount of enzyme that causes an increase in reducing power corresponding to 10 mg of glucose in 30 min, by using soluble starch as a substrate is defined as 1 unit (1 U).

The reaction time, temperature, pH of the reaction solution, and the like for allowing the modified transglucosidase to act on the saccharide-containing raw material are also not particularly limited. The reaction temperature is, for example, 10 to 80°C, preferably 20 to 70°C, more preferably 30 to 60°C. The pH of the reaction solution is, for example, 2 to 9, preferably 3 to 8, more preferably 3.5 to 7. When using the E493V mutant, the pH is preferably 2 to 3. The reaction time is, for example, 30 sec to 48 hr, preferably 1 min to 24 hr, more preferably 10 min to 12 hr or 30 min to 6 hr. When it is desired to increase the production amount of centose, 10 min to 1 hr is preferred. When it is desired to increase the production amount of panose or isomaltose, 1 hr to 6 hr is preferred. By the above reaction conditions, modification of the sugar composition of the saccharide-containing raw material is achieved. These reaction conditions are appropriately selected according to the target saccharide-containing composition. The optimal reaction conditions can be determined through preliminary experiments.

By using the production method of the present invention, a composition or food in which the sugar composition has changed can be produced. One embodiment of the method for producing a saccharide-containing composition of the present invention includes the following steps (1) and (2). In addition, after step (2), a step of inactivating the enzyme may be added.
(1) a step of preparing a saccharide-containing raw material
(2) a step of treating the prepared raw material with the modified transglucosidase

The saccharide-containing composition obtained by the production method of the present invention has a changed sugar composition, and has an improved production ratio of one or more saccharides among panose, centose, isomaltose, and maltotriose, as compared with a saccharide-containing composition obtained by allowing an existing transglucosidase (SEQ ID NO: 1 or 13) to act.

Specific forms of the saccharide-containing composition include isomaltooligosaccharides, foods or beverages, and food or beverage raw materials. Examples of foods or beverages include plant saccharide-containing foods or beverages (plant milk, plant yogurt, and the like). Examples of food or beverage raw materials include plant food or beverage bases and wort.

The modified transglucosidase of the present invention can modify the sugar composition of a saccharide-containing composition. Therefore, the present invention also provides a sugar-composition modifying agent for a saccharide-containing composition, which agent comprising the modified transglucosidase.

### [Example]

### (1. Preparation of Modified Enzyme)

Using, as a template, the nucleotide sequence (SEQ ID NO: 2) of the transglucosidase gene derived from *Aspergillus niger* and introduced into a pUC119 vector, various mutations were introduced, and the following modified transglucosidases were prepared.

| | | |
|---|---|---|
| · | Mutant 1: | G227L mutant of SEQ ID NO: 1 (SEQ ID NO: 3) |
| · | Mutant 2: | G227F mutant of SEQ ID NO: 1 (SEQ ID NO: 4) |
| · | Mutant 3: | G227F/W343Y mutant of SEQ ID NO: 1 (SEQ ID NO: 5) |
| · | Mutant 4: | E493V mutant of SEQ ID NO: 1 (SEQ ID NO: 6) |
| · | Mutant 5: | W343Y mutant of SEQ ID NO: 1 (SEQ ID NO: 7) |

Specifically, PCR was performed by a conventional method and using the following primers and PrimeSTAR (registered trademark) GXL Premix (2×) (manufactured by Takara), thereby introducing mutations.

**[Table 2]**

| | | |
|---|---|---|
| **primer for G227L mutation** | Fw:5' | - CGGATGATCTCACACCTATTGACCAGTCAGTAC -3' **(SEQ ID NO: 25)** |
| | Rv:5' | - GTGTGAGATCATCCGAAGGATATATGGTCAGATTAG -3' **(SEQ ID NO: 26)** |
| **primer for G227F mutation** | Fw:5' | - GGATGATTTCACACCTATTGACCAGTGAGTACTG -3' **(SEQ ID NO: 27)** |
| | Rv:5' | - GGTGTGAAATCATCCGAAGGATATATGGTCAG -3' **(SEQ ID NO: 28)** |
| **primer for W343Y mutation** | Fw:5' | - CAATGTCGTTACGGCTACAACAACTGGTCGG -3' **(SEQ ID NO: 29)** |
| | Rv:5' | - GCCGTAACGACATTGGTGGAATCCAAGAG -3' **(SEQ ID NO: 30)** |
| **primer for E493V mutation** | Fw:5' | - CATGTCTGTCGTTTCATCCTTCTGTGTCGGGAG -3' **(SEQ ID NO: 31)** |
| | Rv: 5' | - GAAACGACAGACATGTCGTACCACACACCATCG -3' **(SEQ ID NO: 32)** |

Using each of the obtained mutant gene products, *E. coli* JM109 was transformed by a conventional method, and gene expression vectors were obtained. The transglucosidase sequences introduced into the gene expression vectors were confirmed. The transformants were shake-cultured in LB medium at 37°C for 8 hr, then subcultured into LB medium, and further shake-cultured at 37°C for 16 hr. Cells were recovered from the culture liquid, and plasmids were recovered from the cells according to a standard method using NucleoBond (registered trademark) Xtra Maxi (manufactured by Takara). The recovered plasmids were cleaved with the restriction enzyme HindIII, and after obtaining the target modified transglucosidase fragment by gel extraction, the target fragment was transformed into *Aspergillus oryzae* using the protoplast-PEG method. The transformants were cultured at 30°C for 4 days in a liquid medium comprising dextrin, yeast extract, and potassium dihydrogen phosphate. The culture liquid was centrifuged, and the centrifuged supernatant was recovered. After treatment with a 0.45 µm membrane filter, desalting and concentration were performed with an ultrafiltration membrane. Thereafter, purification was performed by an anion exchange column, and the resulting product was used as an enzyme sample (Experimental Examples 1 and 2).

The modified transglucosidase gene sequence was cloned into pGAPZαA by a conventional method. *Pichia pastoris* GS115 was transformed by a conventional method with the prepared plasmid. After culturing the transformants under conditions of 30°C, 48 h; BMDY medium (20 g/L Peptone, 10 g/L yeast extract, 3.4 g/L yeast nitrogen base w/o amino acids and ammonium sulfate, 10 g/L ammonium sulfate, 0.1 M potassium phosphate (pH 6.0), 10 g/L glucose, and 0.4 mg/L biotin), enzyme purification was performed by a resin column or the like, and the resulting product was used as an enzyme sample (Experimental Examples 3 and 4).

### (2. Activity Measurement Method)

### <Method for Measuring Transglucosidase Hydrolytic Activity>

The activity of transglucosidase was measured by the following method.
(1) 2.0 g of α-methyl-D-glucoside was weighed and dissolved by adding water to 100 mL to prepare an α-methyl-D-glucoside solution. Into a test tube, 1 mL of the α-methyl-D-glucoside solution and 1 mL of 0.02 mol/L acetic acid/sodium acetate buffer (pH 5.0) were measured, left at 40°C for 10 to 15 min, then 0.5 mL of an aqueous solution comprising transglucosidase (enzyme solution) was added, and the mixture was well shaken. This was left at 40°C for exactly 60 min. Exactly 60 min later, it was placed in a boiling water bath, heated for exactly 5 min, and cooled in running water.
(2) Into a test tube was measured 3 mL of a coloring liquid of Glucose CII-Test Wako (manufactured by Wako Pure Chemical Industries, Ltd.), 0.2 mL of the reaction liquid obtained in (1) was added and well shaken, and then the mixture was left at 40°C for exactly 5 min. For this liquid, absorbance (E60) at a wavelength of 505 nm was measured using water as a control. Separately, as a blank, 1 mL of 0.02 mol/L acetic acid/sodium acetate buffer (pH 5.0) and 0.5 mL of an aqueous solution comprising transglucosidase (enzyme solution) were measured into a test tube, heated in a boiling water bath for exactly 5 min, and then cooled in running water. After cooling, 1 mL of the α-methyl-D-glucoside solution was added, and absorbance (E0) was measured by operating in the same manner as above.
(3) Glucose Standard Solution I or II of Glucose CII-Test Wako was diluted with water to a prescribed concentration (20 mg/dL, 40 mg/dL). Into a test tube was measured 3 mL of Glucose CII-Test Wako coloring liquid, 0.2 mL of each of the above-mentioned glucose solutions was added and well shaken, and then the mixture was left at 40°C for exactly 5 min. For this liquid, absorbance (ES) at a wavelength of 505 nm was measured using water as a control. Separately, as a blank, absorbance (EB) was measured by operating in the same manner as above using 0.2 mL of water instead of the glucose solution. From the obtained absorbance, a calibration curve of glucose was plotted, and the amount of glucose (µg) (G) when the absorbance difference was 1.000 was determined.
(4) The amount of enzyme that produces 1 µg of glucose in 60 min was defined as 1 unit (1 U), and was calculated from the following formula. $Transglucosidase activity \left(U/g, U/mL\right) = \left(\begin{matrix}E 60 - \\ E 0\end{matrix}\right) \times G \times 2.5 / 0.2 \times n / 0.5$ (wherein E60 indicates the absorbance of the reaction liquid, E0 indicates the absorbance of the blank liquid, G indicates the amount of glucose (µg) when the absorbance difference is 1.000, 2.5 indicates the reaction system liquid amount (mL), 0.2 indicates the collected amount of the reaction liquid (mL), 0.5 indicates the collected amount of the enzyme solution (mL), and n indicates the dilution factor per 1 g or 1 mL of the sample.)

### (3. Evaluation of Mutants)

### [Experimental Example 1]

### <Evaluation-1 of Glycosyl Transfer Reaction Using Maltose as Substrate>

Using 3.4% (w/v) maltose/40 mM sodium acetate buffer (pH 4.0) (only for mutant 4, 40 mM glycine-HCl (pH 2.5)) as a substrate, and using a modified transglucosidase at a final concentration of 0.006%, reactions were performed at 37°C for 5 min, 30 min, 1 hr, 2 hr, and 6 hr, and the produced sugars (DP1 to DP3) were analyzed by HPLC-RID using a column TSKgel Amide-80 5 µm 4.6×25 cm. Comparison was made by the ratio of the peak area of each saccharide to the total peak area. Data having the largest production amount in the reactions for 5 min, 30 min, 1 hr, 2 hr, and 6 hr are shown in Tables 3 to 5.

**[Table 3]**

| maltotriose production amount (%) | | |
|---|---|---|
| | reaction time (hr) | maximum production amount |
| reference sequence 1 | 0.08 | 3.7 |
| Mutant 1 | 0.08 | 5.3 |
| Mutant 2 | 0.5 | 5.6 |
| Mutant 3 | 0.08 | 13.5 |

**[Table 4]**

| centose production amount (%) | | |
|---|---|---|
| | reaction time (hr) | maximum production amount |
| reference sequence 1 | 0.08 | 2.4 |
| Mutant 3 | 0.5 | 11.9 |

**[Table 5]**

| panose production amount (%) | | |
|---|---|---|
| | reaction time (hr) | maximum production amount |
| reference sequence 1 | 0.5 | 22.0 |
| Mutant 4 | 6 | 38.6 |

As shown in Tables 3 to 5, when 3.4% maltose was used as a substrate, mutants 1 and 2 were shown to have an increased maximum production amount of maltotriose as compared with the enzyme of reference sequence 1. In addition, mutant 3 was shown to have increased maximum production amounts of maltotriose and centose as compared with the enzyme of reference sequence 1. Furthermore, mutant 4 was shown to have an increased maximum production amount of panose as compared with the enzyme of reference sequence 1.

### [Experimental Example 2]

### <Evaluation-2 of Glycosyl Transfer Reaction Using Maltose as Substrate>

Using 10% (w/v) maltose/40 mM sodium acetate buffer (pH 4.0) (only for mutant 4, 40 mM glycine-HCl (pH 2.5)) as a substrate, and using a modified transglucosidase at a final concentration of 0.006%, reactions were performed at 37°C for 5 min, 30 min, 1 hr, 2 hr, and 6 hr, and the produced sugars (DP1 to DP3) were analyzed by HPLC-RID using a column TSKgel Amide-80 5 µm 4.6×25 cm and a column MCIGEL COLUMN CK04S. Comparison was made by the ratio of the peak area of each saccharide to the total peak area. Data having the largest production amount in the reactions for 5 min, 30 min, 1 hr, 2 hr, and 6 hr are shown in Tables 6 to 9.

**[Table 6]**

| isomaltotriose production amount (%) | | |
|---|---|---|
| | reaction time (hr) | maximum production amount |
| reference sequence 1 | 2 | 26.0 |
| Mutant 2 | 4 | 29.0 |

**[Table 7]**

| maltotriose production amount (%) | | |
|---|---|---|
| | reaction time (hr) | maximum production amount |
| reference sequence 1 | 0.17 | 4.2 |
| Mutant 1 | 0.17 | 8.5 |
| Mutant 2 | 0.17 | 5.6 |
| Mutant 3 | 0.17 | 17.5 |

**[Table 8]**

| centose production amount (%) | | |
|---|---|---|
| | reaction time (hr) | maximum production amount |
| reference sequence 1 | 0.5 | 2.2 |
| Mutant 1 | 5 | 5.0 |
| Mutant 2 | 0.5 | 3.1 |
| Mutant 3 | 2 | 15.3 |
| Mutant 4 | 2 | 1.0 |

**[Table 9]**

| panose production amount (%) | | |
|---|---|---|
| | reaction time (hr) | maximum production amount |
| reference sequence 1 | 0.5 | 31.6 |
| Mutant 4 | 4 | 38.3 |

As shown in Tables 6 to 9, when 10% maltose was used as a substrate, mutant 1 was shown to have increased maximum production amounts of maltotriose and centose as compared with the enzyme of reference sequence 1. In addition, mutant 2 was shown to have increased maximum production amounts of isomaltotriose, maltotriose, and centose as compared with the enzyme of reference sequence 1. Furthermore, mutant 3 was shown to have increased maximum production amounts of maltotriose and centose as compared with the enzyme of reference sequence 1. In addition, mutant 4 was shown to have an increased maximum production amount of panose as compared with the enzyme of reference sequence 1.

### [Experimental Example 3]

### <Initial Velocity Analysis Results>

Using 3.4% (w/v) maltose/40 mM sodium acetate buffer (pH 4.0) (only for mutant 4, 40 mM glycine-HCl (pH 2.5)) as a substrate, 0.05 U/mL of the modified transglucosidase was reacted at 37°C for 2, 4, 8, 12, and 15 min, and the reaction was terminated by maintaining at 100°C for 3 min.

An equal amount of 200 µM sorbitol was added as an internal standard to the mixture after termination, and 20 µL thereof was subjected to HPAEC analysis. A CarboPac PA1 Column (manufactured by Dionex) was used, and elution was performed with 300 mM NaOH (isocratic, 25 min). The column temperature was room temperature, and the flow rate was 0.8 mL/min. Glucose (glc), panose (PN), maltotriose (G3), and centose (2,4-di-O-(α-glucopyranosyl)-glucopyranose, CT) were used as standard sugars to plot calibration curves. The results are shown in Table 10.

**[Table 10]**

| ratio of initial reaction velocity (%) | | | |
|---|---|---|---|
| | maltotriose | panose | centose |
| | α- (1→4 ) transfer | α- (1→6) transfer | α- ( 1→2 ) transfer |
| reference sequence 1 | 20 | 68 | 12 |
| Mutant 1 | 32 | 46 | 22 |
| Mutant 2 | 37 | 39 | 24 |
| Mutant 3 | 60 | 3 | 37 |
| Mutant 4 | 12 | 84 | 3 |

As shown in Table 10, mutants 1, 2, and 3 were shown to have increased ratios of initial reaction velocity of maltotriose and centose as compared with the enzyme of reference sequence 1 when 3.4% maltose was used as a substrate. In addition, mutant 4 was shown to have an increased ratio of initial reaction velocity of panose as compared with the enzyme of reference sequence 1.

### [Experimental Example 4]

### <Evaluation-3 of Glycosyl Transfer Reaction Using Maltose as Substrate>

Using 3.4% (w/v) maltose/40 mM sodium acetate buffer (pH 4.0) as a substrate, 0.05 U/mL of the modified transglucosidase was reacted at 37°C for 3 hr, and the reaction was terminated by maintaining at 100°C for 3 min.

The mixture after termination was analyzed by ion chromatography. The results are shown in Tables 11 to 13.

**[Table 11]**

| 3'-O-α-glucosylmaltose production amount (%) | | |
|---|---|---|
| | reaction time (hr) | maximum production amount |
| reference sequence 1 | 3 | 0.0 |
| Mutant 5 | 3 | 1.1 |
| Mutant 3 | 3 | 7.9 |

**[Table 12]**

| kojibiose production amount (%) | | |
|---|---|---|
| | reaction time (hr) | maximum production amount |
| reference sequence 1 | 3 | 4.7 |
| Mutant 2 | 3 | 8.2 |
| Mutant 5 | 3 | 21.8 |
| Mutant 3 | 3 | 27.9 |

**[Table 13]**

| nigerose production amount (%) | | |
|---|---|---|
| | reaction time (hr) | maximum production amount |
| reference sequence 1 | 3 | 1.0 |
| Mutant 2 | 3 | 2.0 |
| Mutant 5 | 3 | 7.7 |
| Mutant 3 | 3 | 10.8 |

As shown in Tables 11 to 13, when 3.4% maltose was used as a substrate, mutant 2 was shown to have increased maximum production amounts of kojibiose and nigerose as compared with the enzyme of reference sequence 1. In addition, mutants 3 and 5 were shown to have increased maximum production amounts of kojibiose and nigerose as compared with the enzyme of reference sequence 1, and also to produce 3'-O-α-glucosylmaltose, which was not produced by the enzyme of reference sequence 1.

### [Industrial Applicability]

The present invention is extremely useful in various fields using modified transglucosidase, because reaction specificity is modified. This application is based on a patent application No. 2024-011342 filed in Japan (filing date: January 29, 2024), the contents of which are incorporated in full herein.

## Claims

1. A modified transglucosidase consisting of a polypeptide shown in any of the following (1) to (3):
(1) a polypeptide consisting of an amino acid sequence resulting from introduction of at least one of the substitutions (A) to (C):
(A) substitution of the amino acid residue at position 227 with a leucine residue or a phenylalanine residue,
(B) substitution of the amino acid residue at position 343 with a tyrosine residue, and
(C) substitution of the amino acid residue at position 493 with a valine residue
into the amino acid sequence shown in SEQ ID NO: 1 or 13,
(2) a polypeptide consisting of an amino acid sequence into which at least any one substitution shown in the (A) to (C) has been introduced, wherein one or several amino acid residues other than the substituted amino acid residue(s) are substituted, added, inserted, or deleted, and the polypeptide has modified reaction specificity as compared with the polypeptide consisting of the amino acid sequence shown in SEQ ID NO: 1 or 13, and
(3) a polypeptide consisting of an amino acid sequence into which at least any one substitution shown in the (A) to (C) has been introduced, wherein the sequence identity of the portion excluding the substituted amino acid residue(s) is 70% or more, and the polypeptide has modified reaction specificity as compared with the polypeptide consisting of the amino acid sequence shown in SEQ ID NO: 1 or 13.

2. A DNA encoding the modified transglucosidase according to claim 1.

3. An expression cassette or a recombinant vector comprising the DNA according to claim 2.

4. A transformant obtained by introducing the expression cassette or recombinant vector according to claim 3 into a host.

5. A method for producing a modified transglucosidase, comprising a step of culturing the transformant according to claim 4.

6. An enzyme preparation comprising the modified transglucosidase according to claim 1.

7. A method for producing a saccharide-containing composition, comprising a step of allowing the modified transglucosidase according to claim 1 to act on a saccharide-containing raw material.

8. The method according to claim 7, wherein the saccharide-containing composition comprises at least any one selected from the group consisting of maltotriose, isomaltose, panose, centose, isomaltotriose, 3'-O-α-glucosylmaltose, kojibiose, and nigerose.

9. The method according to claim 7 or 8, wherein the saccharide-containing composition is a food or beverage.
